**Europäisches Patentamt**

**European Patent Office**

**Office européen des brevets**

(11) Veröffentlichungsnummer: **0 516 932 A1**

# EUROPÄISCHE PATENTANMELDUNG

(21) Anmeldenummer: **92105261.9**

(22) Anmeldetag: **27.03.92**

(51) Int. Cl.5: **A61F 2/56**, B25J 13/08,
G01L 5/16, G01L 5/22,
G05D 15/01

(30) Priorität: **04.06.91 CH 1652/91**

(43) Veröffentlichungstag der Anmeldung:
**09.12.92 Patentblatt 92/50**

(84) Benannte Vertragsstaaten:
**AT BE CH DE DK ES FR GB GR IT LI LU MC NL PT SE**

(71) Anmelder: **SCHWEIZERISCHE UNFALLVERSICHERUNGSANSTALT SUVA**
**Fluhmattstrasse 1**
**CH-6002 Luzern(CH)**

(72) Erfinder: **Burckhardt, Christof Walter, Prof. Dr.**
**20 avenue du Château**
**CH-1020 Renens(CH)**
Erfinder: **Bierwirth, Wolfgang**
**Gartenweg 11**
**CH-8968 Mutschellen/AG(CH)**

(74) Vertreter: **Kemény, Andreas**
**c/o Kemény AG Patentanwaltbüro Postfach 3414**
**CH-6002 Luzern(CH)**

(54) **Vorrichtung zum Regeln der Greifkraft eines motorisch angetriebenen Greifers, insbesondere für eine Handprothese.**

(57) Es wird das Gleiten zwischen einem durch einen Motor (M) angetriebenen Greifer (HP), insbesondere einer Handprothese (HP), und dem durch den Greifer (HP) ergriffenen Gegenstand (G) vermieden. Dazu ist am Greifer (HP) ein Sensor (SE) vorgesehen, mit dem sowohl die Normalkraft (N) als auch die Tangentialkraft (T) messbar ist, welche zwischen dem Greifer (HP) und dem Gegenstand (G) auftritt. Es sind Vergleichsmittel (MK) vorgesehen, um eine Funktion der beiden genannten Kräfte (N und T) mit einem empirisch ermittelten Gleitschutzsollwert zu vergleichen und die Greifkraft durch den Motor nachzuregeln.

Dadurch dass die zwischen dem Greifer (HP) und dem Gegenstand (G) wirkenden Kräfte gemessen und ausgewertet werden, ist es möglich eine Zwischenlage zwischen dem Greifer (HP) und dem Gegenstand (G) vorzusehen. Also kann beispielsweise bei einem als Handprothese ausgebildeten Greifer (HP) ein ästhetischer Handschuh verwendet werden.

Fig. 6

Fig. 7

Die Erfindung betrifft eine Vorrichtung zum Regeln der Greifkraft eines motorisch angetriebenen Greifers nach dem Oberbegriff des Anspruch 1.

Motorisch angetriebene Greifer, beispielsweise von Industrierobotern oder Handprothesen, können willkürlich zum Ergreifen eines Gegenstands geschlossen oder zum Loslassen des ergriffenen Gegenstands geöffnet werden. Es ist schwierig, die Greifkraft richtig zu bemessen, damit der ergriffene Gegenstand einerseits nichtgleitend festgehalten und andererseits nicht beschädigt wird.

Bei Industrierobotern ist es bekannt, den Beginn des Gleitens eines durch einen Greifer gehaltenen Gegenstands durch den Gegenstand berührende mechanische Tastmittel (z.B. Rolle, Kugel, Taststift) oder mit optischen Mitteln festzustellen. Auch bei Industrierobotern ist das Problem der eventuellen Nachregelung der Greifkraft aktuell.

In der Praxis ist eine Handprothese durch einen elastomeren ästhetischen Handschuh überzogen. Die durch diesen Handschuh bedeckte Handprothese sieht wie eine natürliche Hand aus. Der Handschuh schützt zudem die Mechanik der Handprothese vor äusseren Einflüssen. In der Praxis wird der Handschuh vom Träger alle paar Wochen gewechselt.

Tastmittel an einer Handprothese müssten diesem ästhetischen Handschuh Rechnung tragen, also durch den Handschuh hindurch wirken können, ohne den Handschuh zu beschädigen und ohne den Handschuhwechsel zu erschweren.

Die von Industrierobotern her bekannten mechanischen und optischen Tastmittel zum Erfassen des Gleitbeginns sind daher bei einer Handprothese nicht brauchbar.

Der Erfindung liegt daher die Aufgabe zugrunde, eine Vorrichtung zu schaffen, welche trotz einer Abdeckung des Greifers, wie z.B. des ästhetischen Handschuhs einer Handprothese, das rasche Erfassen des Gleitbeginns und das Einleiten geeigneter Gegenmassnahmen gestattet.

Zur Lösung dieser Aufgabe wird die im Anspruch 1 definierte Vorrichtung vorgeschlagen.

Bei der erfindungsgemässen Vorrichtung misst man also die durch den Greifvorgang hervorgerufene Normalkraft und die für das Vermeiden eines Gleitens des ergriffenen Gegenstands gegenüber dem Greifer hier relevante Tangentialkraft, was auch durch eine Abdeckung (z.B. einen ästhetischen Handschuh einer Handprothese) hindurch erfolgen kann, während man bisher räumliche Veränderungen also Bewegungen gemessen hat, was beim Vorhandensein einer Abdeckung zumindest problematisch ist.

Dazu sind erfindungsgemäss am Greifer Normalkraft-Messmittel vorgesehen, mit denen die Normalkraft zwischen dem Greifer und dem ergriffenen Gegenstand direkt oder durch eine Abdek-

kung hindurch gemessen wird, welche Normalkraft als Folge der durch den Motor erzeugten Greifkraft auftritt.

Ferner sind erfindungsgemäss Normalkraft-Begrenzungsmittel vorgesehen, welche dazu dienen, den Motor abzustellen, wenn die Normalkraft-Messmittel einen vorgegebenen Normalkraft-Grundwert messen, der erfahrungsgemäss eine Beschädigung des ergriffenen Gegenstands ausschliesst.

Ferner sind erfindungsgmäss am Greifer Tangentialkraft-Messmittel vorgesehen, mit denen die Tangentialkraft zwischen dem Greifer und dem ergriffenen Gegenstand direkt oder durch eine Abdeckung hindurch gemessen werden kann, welche Tangentialkraft als Folge des Gewichts und/oder der Masse, insbesondere beim Bewegen des Greifers zum Transportieren des ergriffenen Gegenstands auftreten kann.

Die erfindungsgemässe Vorrichtung hat Vergleichsmittel, mit welchen wenigstens einer der beiden durch die vorgenannten Messmittel gemessenen Kraftwerte direkt oder in umgesetzter Form mit einem vorbestimmten Gleitschutz-Sollwert verglichen wird, wobei gegebenenfalls der Motor durch die Steuermittel eingeschaltet und beim Erreichen geeigneter Sollwerte wieder abgeschaltet wird.

Die genannten Messmittel sind vorzugsweise in Abhängigkeit ihrer mechanischen Belastung in ihren Eigenschaften, insbesondere hinsichtlich ihrer elektrischen und/oder magnetischen Eigenschaften, veränderlich. Man kann dann die Eigenschaftenveränderungen beispielsweise elektronisch einfach und platzsparend verarbeiten.

Besonders bevorzugt werden Messmittel, bei denen sich der elektrische Widerstand in Abhängigkeit ihrer mechanischen Belastung verändert, weil sich solche Messmittel platzsparend und robust ausführen und leicht elektronisch anschliessen lassen. Beispielsweise sind dazu elektrisch leitende Kunststoffe geeignet, die man z.B. mit mäanderartigen Elektroden (gewünschtenfalls als gedruckte Schaltung) versehen kann.

Wenn man bevorzugterweise zwei derartige Widerstände zu einem Sandwich gegenschaltet, lässt sich im relevanten Bereich ein linearer Funktionsverlauf erzielen. Es versteht sich, dass eine lineare Funktion relativ leichter weiterzuverarbeiten ist.

Wenn man zwei derartige Sandwiches nach Art eines sogenannten Joy-Stick mechanisch verbindet und elektrisch schaltet, kann man bevorzugterweise erreichen, dass eine an ihrer mechanischen Verbindung hinsichtlich beider Sandwiches gleichsinnig quer angreifende Normalkraft eine gleichsinnige Veränderung der Widerstände beider Sandwiches hervorruft, während eine auf die mechanische Verbindung hinsichtlich beider Sandwiches entgegengesetzt quer angreifende Tangentialkraft eine in den beiden Sandwiches im entgegengesetzten Sinn verlaufende Widerstandsveränderung hervorruft. Man kann dann die Signale eines so aufgebauten Sensors als Verhältniszahl der beiden Kräfte auswerten und einfach mit entsprechenden empirisch ermittelten Sollwerten vergleichen.

Wenn die Kräfte bei einer erfindungsgemässen Vorrichtung durch eine Greiferabdeckung (beispielsweise durch einen ästhetischen Handschuh einer Handprothese) hindurch gemessen werden, tritt am unter der Abdeckung (dem Handschuh) befindlichen Sensor eine Normalkraft auf. Die Tangentialkraft, welche dann vorerst einmal zwischen der Abdeckung (dem Handschuh) und dem erfassten Gegenstand reibungsbedingt auftritt, kann durch eine dadurch hervorgerufene Bewegung der Abdeckung (des Handschuhs) auf den darunterliegenden Sensor (ebenfalls durch Reibung) übertragen werden. Die Abdeckung, z.B. der ästhetische Handschuh, werden ja in der Regel weitgehend gleichbleibende physikalische Eigenschaften, also auch einen konstanten Reibungskoeffizienten aufweisen, so dass die einmal ermittelten Sollwerte zumindest längere Zeit gültig bleiben könnten. Zudem kann ein automatischer Nullwertabgleich in der elektrischen Schaltung vorgesehen werden.

Im Betrieb kann die erfindungsgemässe Vorrichtung wie folgt funktionieren:

Man setzt den Motor zum Ergreifen eines Gegenstands in Betrieb, wobei am Sensor des Greifers direkt oder durch die Abdeckung eine Normalkraft auftritt, in deren Folge der Sensor ein Signal abgibt. Dieses Signal wird mit einem empirisch ermittelten Grundwert verglichen, der einer Normalkraft entspricht, die zu keinem Schaden am Gegenstand führt. Wird dieser Grundwert erreicht, wird der Motor abgestellt.

Wird nun (beispielsweise durch Armbewegung) der Greifer (z.B. eine Handprothese) bewegt, beispielsweise angehoben, wirkt sich das Gewicht bzw. die Masse des ergriffenen Gegenstands aus, so dass eine Tangentialkraft am Sensor direkt oder durch die Abdeckung (den Handschuh) auftritt, die ein Signal zur Folge hat, das im Verhältnis zur Normalkraft darstellbar und mit einem Sollwert vergleichbar ist. Bei Gleittendenz erreicht dieses Verhältnis einen Gleitschutz-Soll-wert, was zum Wiedereinschalten des Motors und zur Erhöhung der Greifkraft führt. Dadurch wird die auf den Sensor wirkende Normalkraft erhöht, und das Verhältnis zwischen der allenfalls auch veränderten Tangentialkraft und Normalkraft verändert sich, bis ein das Abschalten des Motors bewirkender Sollwert erreicht und der Motor abgestellt wird. Dieser Vorgang kann sich nötigenfalls wiederholen.

Zum Messen der Normalkraft und der Tangentialkraft kommen ausser dem bereits geschilderten die verschiedensten physikalischen Prinzipien in Frage, beispielsweise neben veränderlichen Widerständen auch veränderliche Kapazitäten oder Induktivitäten.

Man kann aus den Signalen des Sensors erhaltene Werte für die Tangentialkraft und für die Normalkraft beispielsweise zur Berechnung des Quotienten der beiden Kräfte nutzen, den man mit empirischen Sollwerten vergleichen und bei entsprechender Differenz zum Ein- und Ausschalten des Antriebs nutzen kann.

Inbesondere bei der Ermittlung der Sollwerte spielt naturgemäss der Reibungskoeffizient zwischen der Abdeckung (dem Handschuh) und dem ergriffenem Gegenstand eine erhebliche Rolle.

Der Reibungskoeffizient zwischen Papier, Glas oder Metall (alle trocken) einerseits und einem üblichen ästhetischen Handschuh einer Prothese andererseits pflegt in der Regel grösser als 0,1 zu sein. Man kann dann das Gleiten verhindern, wenn die Druckkraft F nicht grösser ist als die Reibkraft, die bekanntlich das Produkt aus Reibungskoeffizient (hier z.B. 0,1) und Normalkraft N ist.

Es versteht sich, dass ein so niedriger Reibungskoeffizient, wie er beispielsweise auch bei eingeseiften natürlichen Händen auftreten kann, auch bei Greifern, wie Handprothesen zu Problemen führen kann.

Man kann beispielsweise in einer Handprothese mehrere Sensoren gleichzeitig vorsehen, z.B. einen oder mehrere in den Fingerspitzen (zum Erfassen der Kräfte beim Ergreifen eines Buches und dergleichen), und einen im Inneren der Hand (zum Erfassen der Kräfte beim Ergreifen einer Flasche und dergleichen).

Die von einem oder mehreren Sensoren stammenden Signale werden vorzugsweise mittels eines Mikrokontrollers verarbeitet und nötigenfalls unter Einschaltung weiterer an sich bekannter Mittel zum Ein- und Ausschalten des Motors ausgewertet. Man kann dabei auch ein dem jeweiligen Zweck angepasstes Programm in der Vorrichtung gewünschtenfalls sogar auswechselbar einsetzen.

Die Erfindung wird nachstehend anhand der rein schematischen Zeichnung einer für eine Handprothese geeigneten, z.Zt. bevorzugten Ausführungsform beispielsweise besprochen.

Es zeigen:

Fig. 1 eine schematische Darstellung eines druckabhängig veränderlichen Widerstands,

Fig. 2 ein Druckkraft-Widerstand-Diagramm des Widerstands der Fig. 1,

Fig. 3 eine schematische Darstellung eines Sandwichs aus zwei gegeneinander geschalteten Widerständen gemäss Fig. 1,

Fig. 4 ein Schaltschema des Sandwichs gemäss Fig. 3,

Fig. 5 ein Druckkraft-Widerstand-Diagramm des Sandwichs der Fig. 3,

Fig. 6 eine schematische Darstellung eines Sensors aus zwei Sandwiches gemäss Fig. 3, und

Fig. 7 ein schematisches Schaubild einer Anwendung des in Fig. 6 schematisch dargestellten Sensors.

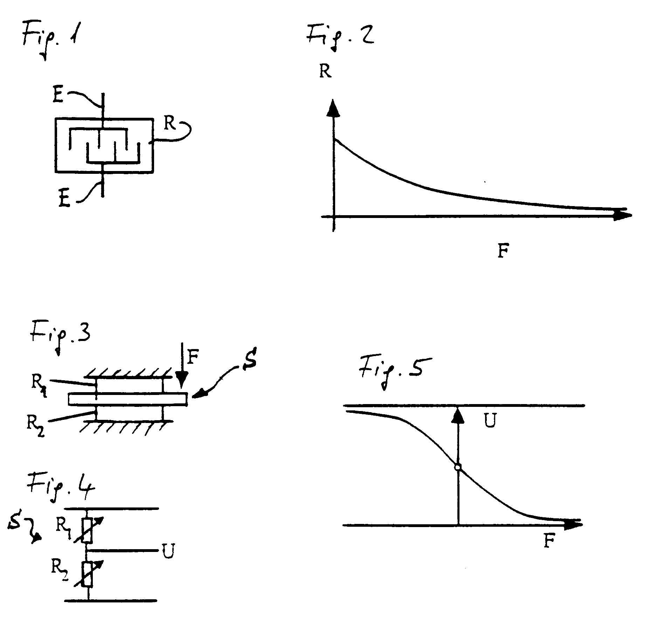

Mit unter Druckeinwirkung ihren elektrischen Widerstand ändernden Kunststoffplättchen R (Fig. 1), auf die man, beispielsweise nach dem Prinzip der gedruckten Schaltungen, Elektroden E aufbrachte, lässt sich, wie Fig. 2 zeigt, mit wachsender Druckkraft F der Widerstand R reduzieren.

Wenn man gemäss Fig. 3 zwei Widerstände R1 und R2 des Bauprinzips der Fig. 1 zu einem Sandwich S gemäss Fig. 4 gegeneinanderschaltet, erhält man, wie Fig. 5 zeigt, eine im relevanten mittleren Bereich linear zum Druck F verlaufende Spannung U.

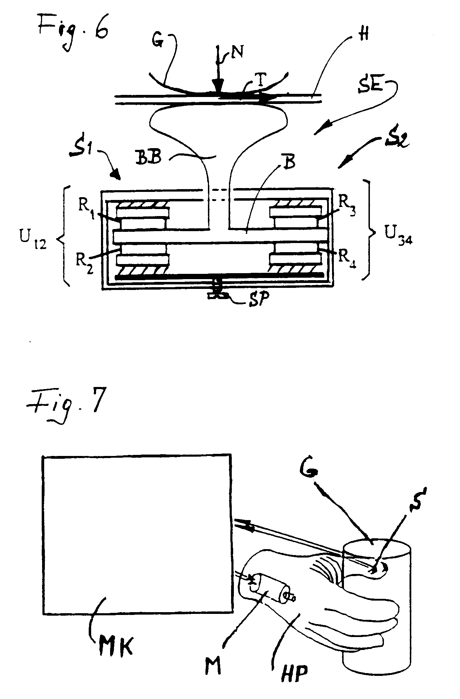

Fig. 6 zeigt einen Sensor S, der durch mechanische Verbindung von zwei solchen Sandwiches S1 (aus R1 und R2) und S2 (aus R3 und R4) mittels eines Balkens B, der einen Tastfortsatz BB hat, erhalten wird. Der Sensor S kann durch Vorspannung, z.B. mittels einer Schraube SP, vorabgestimmt werden.

Dieser Sensor S (Fig. 6) wirkt wie folgt:
- Wirkt eine Normalkraft N auf den Tastfortsatz BB, so ändern sich die Spannungen U12 und U34 im gleichen Sinne, d.h. sie nehmen beide zu, oder sie nehmen beide ab.
- Wirkt eine Tangentialkraft T auf den Tastfortsatz BB, entsteht am Balken B eine ungleiche Wirkung, was auch eine gegensinnige Veränderung der Spannungen U12 und U34 mit sich bringt.
- Dies übrigens unabhängig davon ob zwischen dem Tastfortsatz BB und dem ergriffenen Gegenstand G ein Handschuh H (oder dergleichen) vorhanden ist oder nicht. Lediglich die Reaktion von BB kann beim Vorhandensein oder beim Fehlen des Handschuhs H etwas anders sein, was sich berücksichtigen lässt.

Es gilt dabei folgendes:
Die Normalkraft
N ist proportional U12 + U34.
Die Tangentialkraft
T ist proportional U12 - U 34.

Im Betrieb geschieht folgendes (vgl. Fig. 6 und 7):
- Man führt die geöffnete Handprothese HP, welche mit dem ästhetischen Handschuh H bedeckt ist, zum zu ergreifenden Gegenstand G.
- Man löst willkürlich den Befehl zum Schliessen der Handprothese HP aus.

- Der Motor M schliesst nun die Handprothese HP, welche dabei den Gegenstand G mit einer Greifkraft ergreift.
- In der Folge der Greifkraft entsteht am Sensor S eine Normalkraft N.
- Erreicht diese Normalkraft N einen bestimmten Wert, welcher einer empirisch ermittelten Greifkraft entspricht, bei der der Gegenstand keinen Schaden nimmt, wird der Motor M automatisch angehalten.
- Die Normalkraft N bleibt nun vorerst konstant.
- Wenn man nun die Handprothese HP bewegt, z.B. um den Gegenstand G anzuheben, entsteht eine Tangentialkraft T zwischen Gegenstand G und Handprothese HP.
- In der Folge kann es zum Gleiten des Gegenstands G in der Handprothese HP kommen.
- Dieses beginnende Gleiten führt zu einer Veränderung der Tangentialkraft T zwischen Handprothese HP und Gegenstand G.
- Das wiederum führt zu einer Veränderung des Koeffizienten (U12 - U34) : (U12 + U34), welcher einen empirisch bestimmten Sollwert haben muss, damit kein Gleiten eintritt.
- Wird dieser Sollwert des genannten Koeffizienten nicht erreicht, wird der Motor M automatisch eingeschaltet.
- Das darauf folgende weitere Schliessen der Handprothese HP führt zu einer Erhöhung der Greifkraft, als deren Folge auch die Normalkraft N steigt.
- In der Folge verändert sich auch der genannte Koeffizient, was schliesslich zum Erreichen des Sollwertes führt, worauf der Motor abgeschaltet wird.
- Dieser Vorgang kann sich bei Bedarf wiederholen.

Die Verarbeitung der Signale U12 und U34 erfolgt vorteilhaft mittels eines Mikrokontrollers MK, welcher die Spannungswerte in numerische Werte umsetzt und dann die Berechnung des Quotienten und seinen Vergleich mit dem Sollwert vornimmt, um den Elektromotor M und damit die Handprothese HP im gewünschten Sinne zu steuern.

Weil beispielsweise der leitende Kunststoff der Widerstände R1, R2, R3, R4 gewissen "Langzeit"-Veränderungen unterliegen kann, ist es von Vorteil, wenn der Mikrokontroller MK mit einem automatischen Nullabgleich ausgerüstet ist, der bei Nichtbetätigung des Sensors S die Eingänge der Rechnerstufen auf Null setzt.

Man kann durch die Verwendung weiterer Sandwiches auch eine multidirektionale Einwirkung der Tangentialkraft berücksichtigen.

Wenn man mehrere Sensoren an der Handprothese verwendet, lassen sich geeignete Anpassungen an verschiedene Verwendungen der Handprothese genau berücksichtigen. Das Grundprinzip bleibt in etwa gleich.

**Patentansprüche**

1. Vorrichtung zum Regeln der Greifkraft eines durch einen Motor angetriebenen Greifers, insbesondere für eine Handprothese, mit Erfassungsmitteln zum Feststellen eines drohenden Gleitens bzw. eines Gleitbeginns zwischen dem Greifer und einem durch ihn ergriffenen Gegenstand und mit Steuermitteln zum Nachstellen der Greifkraft durch Ein- und Ausschalten des Motors,
dadurch gekennzeichnet,
dass am Greifer Normalkraft-Messmittel (SE) vorgesehen sind zum Messen einer als Folge der Greifkraft zwischen dem Greifer (HP) und dem ergriffenem Gegenstand (G) auftretenden Normalkraft (N),
und dass Normalkraft-Begrenzungsmittel (MK) vorgesehen sind zum Abstellen des Motors (M) beim Messen eines vorgegebenen Normalkraft-Grundwerts durch die Normalkraft-Messmittel,
und dass ferner am Greifer Tangentialkraft-Messmittel (SE) vorgesehen sind zum Messen einer zwischen dem Greifer (HP) und dem ergriffenen Gegenstand (G) wirkenden Tangentialkraft (T),
und dass ausserdem Vergleichsmittel (MK) vorgesehen sind zum Vergleichen wenigstens eines der beiden durch die vorgenannten Messmittel gemessenen Kraftwerte (N und/oder T und/oder deren Verhältnis) mit einem vorbestimmten Gleitschutz-Sollwert und zum Inbetriebsetzen der genannten Steuermittel.

2. Vorrichtung nach Anspruch 1, dadurch gekennzeichnet, dass die genannten Messmittel (SE) in Abhängigkeit ihrer mechanischen Belastung veränderliche, insbesondere elektrische und/oder magnetische, Eigenschaften aufweisen.

3. Vorrichtung nach Anspruch 2, dadurch gekennzeichnet, dass die Messmittel (SE) einen in Abhängigkeit seiner mechanischen Belastung veränderlichen elektrischen Widerstand (R1, R2, R3, R4) aufweisen.

4. Vorrichtung nach Anspruch 3, dadurch gekennzeichnet, das die Messmittel (SE) einen in Abhängigkeit seiner mechanischen Belastung im relevanten Bereich linear veränderlichen elektrisch Widerstand (S1, S2) aufweisen.

5. Vorrichtung nach Anspruch 4, dadurch gekennzeichnet, dass der genannte elektrische Widerstand (S1, S2) durch paarweises Gegeneinanderschalten in Sandwiches (S1, S2) von druckempfindlichen Einzelwiderständen (R1, R2, R3, R4) aufgebaut ist.

6. Vorrichtung nach Anspruch 5, dadurch gekennzeichnet, dass zwei Sandwiches (S1, S2) dergestalt mechanisch (B, BB) verbunden und elektrisch geschaltet sind, dass eine an ihrer mechanischen Verbindung (B, BB) hinsichtlich beider Sandwiches (S1, S2) gleichsinnig quer angreifende Normalkraft (N) eine gleichsinnige Veränderung der Spannungen (U12, U34) an den Sandwiches (S1, S2) hervorruft, während eine auf die mechanische Verbindung (B, BB) hinsichtlich beider Sandwiches (S1, S2) entgegengesetzt angreifende Tangentialkraft (T) eine in den beiden Sandwiches (S1, S2) im entgegengesetzten Sinn verlaufende Spannungsveränderung (U12, U34) hervorruft.

7. Vorrichtung nach Anspruch 6, dadurch gekennzeichnet, dass Mittel (MK) vorgesehen sind, um den Koeffizient ( (U12 - U 34) : (U12 + U34) ) aus der Differenz der Spannungen (U12 - U 34) und der Summe der Spannungen (U12 + U34) mit einem empirisch ermittelten Sollwert zu vergleichen.

Fig. 1

Fig. 2

Fig. 3

Fig. 4

Fig. 5

Fig. 6

Fig. 7

Europäisches
Patentamt

EUROPÄISCHER RECHERCHENBERICHT

Nummer der Anmeldung

EP    92 10 5261
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | GB-A-2 163 570 (J. E. HANGER &CO LTD) * Seite 1, Zeilen 3 - 33; Seite 1, Zeilen 69 - 109; Seite 1, Zeile 122 - Seite 2, Zeile 30; Figuren 1, 2; Anspruch 1 * --- | 1,2 | A61F2/56 B25J13/08 G01L5/16 G01L5/22 G05D15/01 |
| A | US-A-3 509 583 (A. V. FRAIOLI) * Spalte 1, Zeilen 19 - 35; Spalte 2, Zeile 51 - Spalte 3, Zeile 4; Spalte 3, Zeile 40 - Spalte 4, Zeile 13; Spalte 4, Zeile 54 - Spalte 5, Zeile 23; Spalte 5, Zeile 43 - Spalte 6, Zeile 24 ; Figuren 1 - 4 * --- | 1-4 | |
| A | EP-A-0 133 997 (HITACH LTD.) * Zusammenfassung; Seite 1, Zeilen 1 - 4; Seite 1, Zeile 18 - Seite 2, Zeile 1; Seite 5, Zeile 2 - Seite 10, Zeile 5; Figuren 1 - 8 * --- | 2-6 | |
| A | IEEE INTERNATIONAL CONFERENCE ON ROBOTICS AND AUTOMATION, Bd. 1, 15. Mai 1989, THE REGISTRY RESORT, SCOTTSDALE, AZ Seiten 302 - 307; A. BICCHI ET AL.: 'AUGMENTATION OF GRASP ROBUSTNESS USING INTRINSIC TACTILE SENSING' * Zusammenfassung; Kapitel 1. "INTRODUCTION"; Kapitel 2. "SIMPLE PLANAR GRASP" ; Figuren 1 - 4 * --- | 1,2,7 | |
| A | DE-A-3 332 147 (SIEMENS AG) * ZUsammenfassung; Seite 6, Zeile 6 - Seite 8; Zeile 8; Seite 10, Zeilen 8 - 22; Figuren 1, 2, 7 * --- | 1-4,7 | |
| A | GB-A-2 223 315 (BRUNEL UNIVERSITY) * Seite 1, Zeilen 3 - 16; Seite 3, Zeile 1 - Seite 5, Zeile 18; Figuren 1 - 3 * --- -/-- | 1,2 | |

**RECHERCHIERTE SACHGEBIETE (Int. Cl.5 )**

A61F
B25J
G01L
G05D

Der vorliegende Recherchenbericht wurde für alle Patentansprüche erstellt

| Recherchenort | Abschlußdatum der Recherche | Prüfer |
|---|---|---|
| BERLIN | 04 SEPTEMBER 1992 | BEITNER M. |

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | GB-A-2 115 935 ( LORD CORP. ) <br> * Zusammenfassung; Seite 3, Zeilen 31 - 117; Seite 4, Zeilen 41 - 80; Seite 5, Zeilen 80 - 98; Seite 6, Zeilen 8 - 61; Seite 7, Zeilen 26 - 56; Figuren 1, 2, 5, 7 * <br><br> ----- | 1,2 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| BERLIN | 04 SEPTEMBER 1992 | BEITNER M. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
...............................................................................
& : Mitglied der gleichen Patentfamilie, übereinstimmendes
    Dokument

EPO FORM 1503 03.82 (P0403)